# FASCICULE DE BREVET EUROPEEN

(11) **EP 3 237 577 B1**
(45) Date de publication et mention de la délivrance du brevet: **04.09.2019**
(21) Numéro de dépôt: 15817222.1
(22) Date de dépôt: 17.12.2015
(51) Int. Cl.: C10G 47/00, C10G 7/00

(54) **PROCÉDÉ ET DISPOSITIF DE RÉDUCTION DES COMPOSÉS AROMATIQUES POLYCYCLIQUES LOURDS DANS LES UNITÉS D'HYDROCRAQUAGE**
VERFAHREN UND VORRICHTUNG ZUR REDUZIERUNG VON SCHWEREN POLYCYCLISCHEN AROMATISCHEN VERBINDUNGEN IN EINHEITEN ZUM HYDROCRACKEN
PROCESS AND APPARATUS FOR REDUCING HEAVY POLYCYCLIC AROMATIC COMPOUNDS IN HYDROCRACKING UNITS

(30) Priorité: 22.12.2014 FR 1463097
(43) Date de publication de la demande: 01.11.2017
(73) Titulaire: AXENS, 92508 Rueil-Malmaison Cedex (FR)
(72) Inventeur: SAUGE, Thibault, 92500 Rueil-Malmaison (FR); GONZALEZ LLAMAZARES, Roberto, Londres Greater London SW49SL (GB); BONNARDOT, Jérôme, 78330 Fontenay le Fleury (FR); FRECON, Jacinthe, 92500 Rueil-Malmaison (FR)
(74) Mandataire: IFP Energies nouvelles
(86) Numéro de dépôt international: PCT/EP2015/080213
(87) Numéro de publication internationale: WO 2016/102298

(56) Documents cités:
- EP-A2- 0 665 283
- US-A- 2 934 492
- US-A- 3 494 861
- US-A- 3 655 551
- US-A1- 2009 065 401

## Description

L'invention concerne un procédé et un dispositif permettant de réduire la concentration en composés aromatiques polycycliques lourds (HPNA) dans la boucle de recyclage des unités d'hydrocraquage.

Les procédés d'hydrocraquage sont couramment utilisés en raffinerie pour transformer des mélanges hydrocarbonés en produits aisément valorisables. Ces procédés peuvent être utilisés pour transformer des coupes légères telles que par exemple des essences en coupes plus légères (LPG). Ils sont toutefois habituellement plutôt utilisés pour convertir des charges plus lourdes (telles que des coupes pétrolières ou synthétiques lourdes, par exemple des gasoils issus de distillation sous vide ou des effluents d'une unité Fischer-Tropsch) en essence ou naphta, kérosène, gasoil. Ce type de procédé est également utilisé pour produire des huiles.

Afin d'augmenter la conversion des unités d'hydrocraquage, une partie de la charge non convertie est recyclée soit dans la section réactionnelle dans laquelle elle est déjà passée soit dans une section réactionnelle indépendante. Ceci induit une accumulation indésirable des composés aromatiques polycycliques, formés dans la section réactionnelle durant les réactions de craquage, dans la boucle de recyclage. Ces composés empoisonnent le catalyseur d'hydrocraquage, ce qui réduit l'activité catalytique ainsi que la durée de cycle. Ils peuvent également précipiter ou se déposer dans les parties froides de l'unité, générant ainsi des dysfonctionnements.

Il y a donc nécessité d'améliorer le procédé d'hydrocraquage afin de diminuer la formation des composés aromatiques polycycliques, ou de les éliminer sans diminuer le rendement en produits valorisables.

Les composés HPNA sont définis comme des composés aromatiques polycycliques ou polynucléaires qui comprennent donc plusieurs noyaux ou cycles benzéniques condensés. On les nomme habituellement HPA, Heavy Polynuclear Aromatics selon la terminologie anglo-saxonne, ou PNA ou HPNA.

Typiquement, les HPNA dits lourds comprennent au moins 4, voire au moins 6 cycles benzéniques dans chaque molécule. Les composés à moins de 6 cycles (dérivés du pyrène par exemple) peuvent être plus facilement hydrogénées et sont donc moins susceptibles d'empoisonner des catalyseurs. En conséquence nous nous sommes plus particulièrement intéressés aux composés les plus représentatifs des familles à 6 cycles aromatiques ou plus comme par exemple le Coronène (composé à 24 carbones), le dibenzo(e,ghi) pérylène (26 carbones), le naphto[8,2,1,abc] coronène (30 carbones) et l'ovaléne (32 carbones), qui sont les composés les plus facilement identifiables et quantifiables par exemple par chromatographie.

Le brevet US 7,588,678 de la demanderesse décrit un procédé d'hydrocraquage avec recyclage de la fraction non convertie 380°C+, procédé dans lequel les composés HPNA sont éliminés de la fraction recyclée au moyen d'un adsorbant. D'autres techniques de réduction de la quantité ou d'élimination des HPNA sont décrites dans l'art antérieur de ce brevet, telles que par exemple leur réduction via une hydrogénation ou leur précipitation suivie d'une filtration.

Le brevet US 4,961,839 décrit un procédé d'hydrocraquage permettant d'augmenter la conversion par passe en utilisant des débits d'hydrogène élevés dans la zone réactionnelle, en vaporisant une proportion importante des hydrocarbures envoyés dans la colonne de séparation des produits et en concentrant les composés aromatiques polycycliques dans une petite fraction lourde qui est extraite de cette colonne. Dans ce procédé, une fraction lourde est soutirée au niveau d'un plateau situé au-dessus du point d'alimentation et en-dessous du point de soutirage du distillat diesel ; cette fraction lourde est recyclée vers l'hydrocraquage. Le fond de colonne (résidu) est recyclé directement dans la colonne de fractionnement. Ce type de technique permet certes une diminution de la concentration en HPNA dans la boucle de recyclage vers le réacteur, mais conduit à des pertes de rendements significatives et des coûts importants liés aux quantités d'hydrogène.

Les demandes de brevet et WO 2012/052042 et WO 2012/052116 (correspondant à US-2013/0220885) décrivent un procédé d'hydrocraquage dans lequel le fond de la colonne de fractionnement (résidu) est strippé à contre-courant dans une colonne de stripage. La fraction légère obtenue après stripage est renvoyée à la colonne de fractionnement et la fraction lourde issue du stripage est au moins en partie purgée, l'autre partie de cette fraction pouvant être recyclée à la colonne de stripage.

Ces procédés ont apporté des améliorations dans la réduction des HPNA mais souvent au détriment des rendements et des coûts.

Les brevets US 3,655,551, EP 0 665 283, US 3,494,861, US 2,934,492 et US 2009/0065401 décrivent un procédé d'hydrocraquage.

Le procédé de l'invention permet non seulement de concentrer les hydrocarbures aromatiques polycycliques dans des fractions non converties (résidus) afin de les éliminer et de réduire la quantité de résidu purgée pour augmenter la conversion, mais aussi d'améliorer le rendement en produits valorisables (par exemple en évitant le sur-craquage du diesel) et/ou la durée de cycle catalytique par rapport aux procédés antérieurs. L'invention a également pour avantage de réduire considérablement la quantité présente en hydrocraquage de HPNA ayant au moins 6 cycles aromatiques, qui sont les plus réfractaires aux réactions mises en jeu lors de l'hydrocraquage.

Le procédé selon l'invention est basé sur la mise en place d'un soutirage latéral au-dessus de l'alimentation de la colonne, et en-dessous du soutirage du distillat gasoil le plus lourd, avec renvoi dans la colonne de tout ou partie de ladite fraction soutiré, éventuellement séparée du liquide. Une sur-vaporisation est ainsi produite. La séparation du liquide a lieu de préférence en associant un stripeur à la colonne de fractionnement, qui strippe ladite fraction soutirée.

Plus précisément, l'invention concerne un procédé d'hydrocraquage d'une charge pétrolière comprenant au moins 10% volume de composés bouillant au-dessus de 340°C, comprenant une étape d'hydrocraquage, éventuellement suivie d'une séparation des gaz de l'effluent hydrocraqué, puis d'une étape de fractionnement dudit effluent, qui sépare au moins un distillat et un résidu, ledit résidu étant en partie recyclé vers l'étape d'hydrocraquage et une autre partie du résidu étant purgée, ladite étape de fractionnement comporte une distillation dans une colonne munie de plateaux, colonne dans laquelle :
- ledit effluent au moins partiellement vaporisé alimente la colonne sur au moins un plateau d'alimentation
- ledit distillat est soutiré au niveau d'un plateau de soutirage,
- ledit résidu est évacué à un point d'évacuation,
- et, éventuellement, un gaz de stripage est injecté à un point d'injection situé en-dessous du plateau d'alimentation,
   procédé dans lequel
- il est soutiré de la colonne une partie du flux présent au niveau d'au moins un plateau situé entre ledit plateau d'alimentation et le plateau de soutirage de la fraction distillat la plus lourde, ledit au moins un plateau étant situé au-dessus dudit plateau d'alimentation et en-dessous dudit plateau de soutirage de la fraction distillat la plus lourde,
- et une partie dudit flux soutiré est recyclée dans la colonne, directement ou après une éventuelle séparation du liquide,
- et l'autre partie dudit flux soutiré est recyclée dans l'étape d'hydrocraquage directement ou après une éventuelle séparation des gaz.

De façon avantageuse, il est soutiré de la colonne une partie du flux présent au niveau d'un plateau situé au-dessus du plateau d'alimentation et proche dudit plateau d'alimentation, et de préférence au niveau du plateau le plus proche du plateau d'alimentation.

Ledit flux soutiré présente une concentration en HPNA inférieure à 500 ppm poids, préférentiellement inférieure à 350 ppm poids et très préférentiellement inférieure à 200ppm poids. Il présente le plus souvent une proportion d'au moins 70%poids en hydrocarbures non convertis, préférentiellement d'au moins 80%poids en hydrocarbures non convertis et très préférentiellement d'au moins 90%poids en hydrocarbures non convertis.

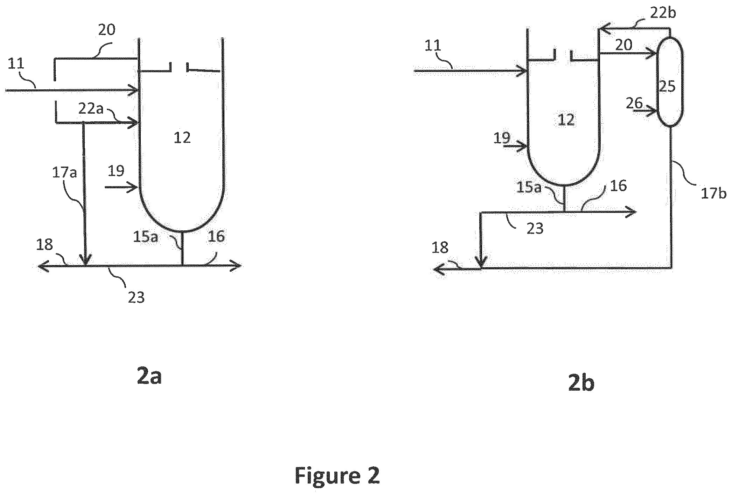

Selon un mode de réalisation du procédé selon l'invention :
- une partie dudit flux soutiré est recyclée directement (i.e. sans traitement du flux soutiré avant recyclage dans la colonne) dans la colonne, par exemple tel que montré sur la figure 2a.
- l'autre partie du flux soutiré est recyclée vers l'étape d'hydrocraquage, directement ou après une éventuelle séparation des gaz.

Avantageusement, ledit flux (en totalité ou en partie) est recyclé dans la colonne au-dessous du plateau d'alimentation et, lorsqu'il existe, au-dessus du point d'injection du gaz de stripage. De façon très préférée, de plus, il est recyclé dans la colonne au niveau du plateau le plus proche dudit plateau d'alimentation

Selon un autre mode de réalisation préféré du procédé selon l'invention :
- tout ou partie, et de préférence la totalité, dudit flux soutiré est séparé dans une étape de stripage externe par un gaz de stripage, et
- tout ou partie, et de préférence la totalité, de l'effluent gazeux séparé est recyclé dans la colonne au-dessus du plateau duquel le flux a été soutiré, et de préférence au niveau d'un plateau proche du plateau duquel le flux a été soutiré,
- préférentiellement, de plus, l'effluent gazeux est recyclé dans la colonne au niveau du plateau le plus proche du plateau duquel le flux a été soutiré.
- préférentiellement, de plus, tout ou partie, et de préférence la totalité, de l'effluent liquide séparé dans ladite étape de stripage est recyclé, de préférence directement, dans l'étape d'hydrocraquage.

Par ailleurs, dans une disposition préférée de ce mode de réalisation, il n'y a pas de recyclage de la fraction liquide, séparée à l'étape de stripage, vers la colonne de fractionnement.

On notera également que selon l'invention, de préférence, il n'y a pas de recyclage de résidu dans l'étape de fractionnement.

De préférence, le procédé opère en présence d'un gaz de stripage injecté dans l'étape de fractionnement. De préférence, c'est de la vapeur d'eau, de préférence à une pression comprise entre 0,2 et 1,5MPa.

Le gaz de stripage injecté dans l'étape de stripage externe est de préférence de la vapeur d'eau, de préférence à une pression comprise entre 0,2 et 1,5MPa.

L'étape d'hydrocraquage a lieu, de façon classique, à une température supérieure à 200°C, une pression supérieure à 1MPa, une vitesse spatiale de 0,1 à 20h⁻¹, et le rapport volumique H2/hydrocarbures est de 80 à 5000NI/l.

L'invention concerne également une installation qui est avantageusement mise en oeuvre pour réaliser le procédé selon l'invention.

Elle comprend :
- une section d'hydrocraquage (2) munie d'une ligne (1) d'entrée de la charge et d'une ligne (8) d'entrée de l'hydrogène,
- éventuellement suivie d'une zone de séparation (4) de l'effluent (ligne 3) pour séparer une fraction gazeuse (ligne 5),
- suivie d'une section de fractionnement (12) comprenant au moins une colonne de distillation munie de plateaux, ladite colonne comportant :
   - au moins une ligne (11) d'entrée de l'effluent hydrocraqué au moins partiellement vaporisé sur au moins un plateau d'alimentation
   - au moins une ligne (14) pour le soutirage d'au moins un distillat au niveau d'un plateau de soutirage,
   - au moins une ligne (15a) d'évacuation du résidu,
- au moins une ligne (23,18) pour le recyclage d'une partie du résidu dans l'étape d'hydrocraquage,
- et comprenant éventuellement au moins une ligne (19) pour l'injection d'un gaz de stripage, le point d'injection étant situé en-dessous du plateau d'alimentation,
installation comprenant en outre :
- au moins une ligne (20) pour le soutirage d'une partie du flux présent au niveau d'au moins un plateau situé, entre ledit plateau d'alimentation et le plateau de soutirage de la fraction distillat la plus lourde, ledit au moins un plateau étant situé au-dessus dudit plateau d'alimentation et en-dessous dudit plateau de soutirage de la fraction distillat la plus lourde,
- et une ligne de recyclage dans ladite colonne, directement (ligne 22a) ou après une éventuelle séparation du liquide (ligne 22b), d'une partie dudit flux soutiré,
- et une ligne de recyclage dans la section d'hydrocraquage de l'autre partie dudit flux soutiré, directement (ligne 17a) ou après une éventuelle séparation des gaz (ligne 17b).

De préférence, le plateau de soutirage dudit flux de la ligne (20) est positionné au niveau d'un plateau proche du plateau d'alimentation. De façon très préférée, le plateau de soutirage dudit flux de la ligne (20) est positionné au niveau du plateau le plus proche du plateau d'alimentation.

Dans un mode de réalisation, l'installation ne comporte pas de traitement du flux soutiré avant recyclage dans la colonne ; elle comporte une ligne (22a) de recyclage directement dans la colonne (i.e. absence de traitement du flux soutiré avant recyclage dans la colonne).

Dans un mode de réalisation, l'installation ne comporte pas de traitement du flux soutiré avant recyclage dans la colonne, elle comporte :
- une ligne (22a) de recyclage directement dans la colonne d'une partie du flux soutiré, et
- une ligne de recyclage (17a) directement dans la section d'hydrocraquage (2) d'une partie du flux soutiré
- avantageusement, la ligne (22a) débouche au niveau d'un plateau situé en-dessous du plateau d'alimentation et, lorsqu'il existe, au-dessus du point d'injection du gaz de stripage (19), et de préférence au niveau d'un plateau proche du plateau d'alimentation,
- de préférence, la ligne (22a) débouche au niveau du plateau inférieur le plus proche du plateau d'alimentation.

Dans un autre mode de réalisation préféré, l'installation selon l'invention comporte :
- un stripeur (25) externe à la colonne, muni d'une ligne (20) d'entrée dudit flux soutiré, d'une ligne (26) d'injection du gaz de stripage, d'une ligne (22b) de sortie de la fraction gazeuse, d'une ligne (17b) de sortie de la fraction liquide,
- une ligne (22b) de recyclage de tout ou partie, et de préférence de la totalité, de ladite fraction gazeuse dans ladite colonne,
- une ligne (17b, 18) de recyclage de tout ou partie, et de préférence de la totalité, de ladite fraction liquide dans l'étape d'hydrocraquage,
- une ligne (16) pour la purge d'une partie du résidu,
- de préférence, la ligne (22b) de recyclage débouche dans la colonne au-dessus du plateau duquel le flux a été soutiré, et de préférence au niveau d'un plateau proche du plateau duquel le flux a été soutiré, et de préférence au niveau du plateau le plus proche du plateau duquel le flux a été soutiré.

Dans une disposition préférée de ce mode de réalisation, il n'y a pas de ligne de recyclage de la fraction liquide, séparée à l'étape de stripage, vers la colonne de fractionnement.

De façon préférée, l'installation selon l'invention ne comporte pas de ligne de recyclage du résidu dans la colonne.

Dans le texte, on définit les charges par leur point d'ébullition T5 (comme cela est expliqué plus loin). La conversion de la charge est définie par rapport au point de coupe du résidu. La fraction non convertie est appelée résidu. La fraction convertie comprend les fractions recherchées (objectifs) par le raffineur.

L'invention sera mieux comprise à partir de la description des figures.

La partie purgée se réfère à une partie qui sort du procédé.

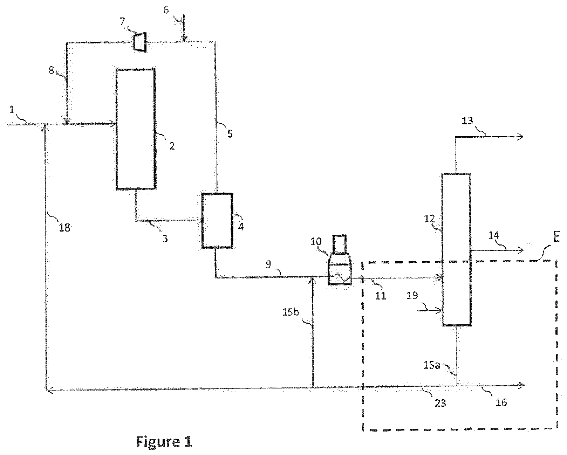

La figure 1 représente l'art antérieur. Les figures 2a et 2b représentent l'invention. Le principe de l'invention sera explicité à partir de la figure 2a. Les figures 2a et 2b se comprennent en combinaison avec la figure 1, et plus précisément avec les éléments essentiels de la figure 1 cités dans les revendications.

La figure 1 présente un schéma de procédé d'hydrocraquage selon l'art antérieur. Pour faciliter la lecture, on a reporté la description des conditions de mise en oeuvre plus loin dans le texte.

La charge (ligne 1) composée d'hydrocarbures d'origine pétrolière et/ou d'hydrocarbures synthétiques de source minérale ou biologique est mélangée à de l'hydrogène alimenté par les lignes 5 (recycle) et/ou 6 (hydrogène d'appoint) via le compresseur 7 et la ligne 8. Le mélange charge/hydrogène ainsi réalisé est envoyé dans la section d'hydrocraquage 2. Cette section comporte un ou plusieurs réacteurs en lit fixe ou en lit bouillonnant.

Lorsque la section d'hydrocraquage comprend un ou plusieurs réacteurs en lit fixe, chaque réacteur peut comprendre un ou plusieurs lits de catalyseur réalisant l'hydrocraquage des hydrocarbures de la charge en hydrocarbures plus légers.

Lorsque la section d'hydrocraquage comprend un ou plusieurs réacteurs en lit bouillonnant, un flux, comprenant du liquide du solide et du gaz, circule verticalement à travers un réacteur contenant un lit de catalyseur. Le catalyseur dans le lit est maintenu en mouvement aléatoire dans le liquide. Le volume brut du catalyseur dispersé à travers le liquide est donc supérieur au volume du catalyseur à l'arrêt. Cette technologie est largement décrite dans la littérature.

Un mélange de liquide hydrocarboné et d'hydrogène est passé à travers le lit de particules de catalyseur à une vitesse telle que les particules sont misent en mouvement aléatoire et donc en suspension dans le liquide. L'expansion du lit catalytique dans la phase liquide est contrôlée par le débit de liquide de recyclage de façon à ce que à l'état d'équilibre, la majeure partie du catalyseur ne dépasse pas un niveau définit dans le réacteur. Les catalyseurs sont sous forme d'extrudés ou de billes, de préférence de diamètre compris entre 0,8 mm et 6,5 mm de diamètre. Dans un procédé en lit bouillonnant des quantités importantes de gaz d'hydrogène et de vapeurs d'hydrocarbures légers montent à travers la zone réactionnelle puis dans une zone exempte de catalyseur. Le liquide provenant de la zone catalytique est pour partie recyclé dans le fond du réacteur après séparation d'une fraction gazeuse et pour partie retiré du réacteur en tant que produit, le plus souvent en partie haute du réacteur.

Les réacteurs utilisés dans un procédé en lit bouillonnant sont généralement conçus avec un conduit de recyclage vertical central qui sert de tube d'écoulement pour le recyclage de liquide de la zone exempte de catalyseur située au-dessus du catalyseur en lit bouillonnant, via une pompe de recyclage qui permet de recycler le liquide dans la zone catalytique. Le recyclage de liquide permet à la fois de maintenir l'uniformité de la température dans le réacteur et de maintenir le lit de catalyseur en suspension.

La section d'hydrocraquage peut être précédée ou inclure un ou plusieurs lits de catalyseur(s) d'hydrotraitement.

L'effluent de la section d'hydrocraquage (2) est envoyé par ligne (3) vers une zone de séparation (4) permettant de récupérer d'une part une fraction gazeuse (5) et une fraction liquide (9). La fraction gazeuse (5) contient l'hydrogène en excès qui n'a pas réagi dans la section réactionnelle (2). Elle est généralement combinée avec de l'hydrogène frais arrivant par la ligne (6) afin d'être recyclée comme indiqué ci-avant.

La fraction liquide (9) est réchauffée par tout moyen (10), par exemple un four éventuellement associé à un échangeur (non représenté), afin d'être au moins en partie vaporisée, avant d'alimenter la section de fractionnement (12) via la ligne (11).

La section de fractionnement (12) comprend une ou plusieurs colonnes de distillation équipées de plateaux et d'internes permettant de séparer différentes coupes (distillats) valorisables qui sont soutirées au moyen des lignes (13) et (14), plus éventuellement d'autres soutirages latéraux. Ces coupes présentent des gammes de points d'ébullition situés par exemple dans la gamme des essences, du kérosène et du gasoil.

En fond de colonne on récupère une fraction plus lourde non convertie (résidu) (ligne 15a).

Une injection de gaz de stripage peut être prévue via la ligne (19). Cette ligne est située entre le plateau d'alimentation en effluent hydrocraqué (ligne 11) et le point d'évacuation du résidu (ligne 15a).

Une partie du résidu peut être purgée via la ligne (16), une autre partie recyclée vers la section d'hydrocraquage par les lignes (23) et (18) et une autre partie recyclée vers la section de fractionnement (ligne 15b).

Selon la figure 1, une partie (ligne 15b) du résidu de la ligne (15a) est mélangée à l'alimentation (ligne 9) en amont du four (10) de la section de fractionnement et recyclée en mélange (ligne 11) vers la section de fractionnement.

La purge (16) permet notamment d'éliminer au moins en partie les composés HPNA qui sans cette purge pourraient s'accumuler dans la boucle de recyclage.

La zone E tracée sur la figure 1 délimite la partie modifiée dans le cadre de la présente invention.

Les figures 2a et 2b présentent l'invention.

On ne reprendra pas les éléments décrits précédemment. On notera que la ligne (15b) (recyclage du résidu à la colonne de fractionnement) est préférentiellement supprimée dans l'invention.

La section de fractionnement (12) comprend une seule colonne de fractionnement. Toutefois, l'invention pourrait-être réalisée avec plusieurs colonnes de fractionnement et au moins une colonne comprendrait alors une zone E selon l'invention.

Selon la figure 2a, la fraction liquide (11) qui a été préalablement au moins en partie vaporisée alimente la section de fractionnement (12).

De préférence, un gaz de stripage est injecté dans la colonne (ligne 19). Avantageusement, il s'agit de vapeur, de préférence de la vapeur basse pression, de préférence à une pression comprise entre 0,2 et 1,5 MPa (0,1MPa=1bar ou 1barg). Le point d'injection est situé en-dessous du plateau d'alimentation et au-dessus du point d'évacuation du résidu. Il est de préférence proche du point d'évacuation du résidu en fond de la colonne.

La figure 2a se distingue de la figure 1 notamment en ce qu'on ajoute un soutirage latéral (ligne 20) au niveau d'un des plateaux de la colonne. On peut mettre en place un ou plusieurs soutirages au niveau de la colonne. Il est ainsi soutiré une partie du flux présent au niveau d'au moins un plateau situé entre le plateau d'alimentation en effluent et le plateau de soutirage (ligne 14) de la fraction distillat la plus lourde.

Ce soutirage (ligne 20) est de préférence proche du plateau d'alimentation. De préférence, il est soutiré de la colonne une partie du flux présent au niveau du plateau le plus proche du plateau d'alimentation.

Tout ou partie dudit flux soutiré est recyclé directement dans la colonne (ligne 22a).

La figure 2b (décrite plus loin) décrit un autre mode de réalisation dans lequel le recyclage dans la colonne a lieu après séparation du liquide.

La figure 2a présente le cas où une partie du soutirage latéral (ligne 20) est recyclée à la colonne (ligne 22a), l'autre partie du flux soutiré (ligne 17a) est recyclée (ligne 18) vers l'étape d'hydrocraquage, de préférence en mélange avec la partie du résidu recyclée (ligne 23) vers l'étape d'hydrocraquage.

Le soutirage latéral (ligne 20) est positionné de manière à ce que le flux soutiré présente une faible concentration en HPNA inférieure à 500 ppm poids, préférentiellement inférieure à 350 ppm poids et très préférentiellement inférieure à 200 ppm poids, et, le plus souvent, une proportion importante d'hydrocarbures non convertis dans la section d'hydrocraquage d'au moins 70 % poids en hydrocarbure non convertis, préférentiellement d'au moins 80%poids en hydrocarbures non convertis et très préférentiellement d'au moins 90%poids en hydrocarbures non convertis.

Afin de respecter ces critères, le soutirage (ligne 20) est préférentiellement positionné au-dessus du plateau d'alimentation, et de préférence au niveau du plateau le plus proche du plateau d'alimentation.

Dans ce mode de réalisation (tel que figure 2a), le flux soutiré est ré-injecté au-dessous du plateau d'alimentation et, lorsqu'il existe, au-dessus du point d'injection du gaz de stripage, et préférentiellement au niveau du plateau le plus proche du plateau d'alimentation.

Pour la description de la figure 2b, on ne décrira pas à nouveau les références des figures 1 et 2a. La figure 2b diffère de la figure 2a sur le traitement et le recyclage du flux soutiré.

Selon la figure 2b, tout ou partie dudit flux soutiré est recyclé dans la colonne après une séparation du liquide.

De façon préférée, le flux soutiré (ligne 20) est strippé dans une étape de stripage externe (stripeur 25) par un gaz de stripage (amené par la ligne 26). Tout ou partie de l'effluent gazeux séparé est recyclé dans la colonne ; selon la figure 2b, la totalité de l'effluent gazeux est recyclé (22b).

Le soutirage latéral (ligne 20) est positionné de la même façon que sur la figure 2a.

De préférence, l'effluent gazeux est recyclé dans la colonne au-dessus du plateau duquel le flux a été soutiré. De plus, de meilleures performances sont obtenues lorsque l'effluent gazeux est recyclé dans la colonne au niveau du plateau le plus proche du plateau duquel le flux a été soutiré.

Tout ou partie de l'effluent liquide (ligne 17b) est recyclé, le plus souvent directement, dans l'étape d'hydrocraquage. Selon la figure 2b, tout l'effluent liquide est mélangé avec la partie du résidu recyclée (ligne 23) et le mélange est recyclé (ligne 18) vers l'étape d'hydrocraquage.

Ledit stripeur latéral 25 fonctionne avec injection d'un gaz de stripage (ligne 26). Ce gaz est de préférence de la vapeur, préférentiellement de la vapeur basse pression, de préférence à une pression comprise entre 0,2 et 1,5MPa.

Ainsi que le montrent les exemples ci-après, le mode de réalisation de la figure 2b conduit à des performances meilleures que le mode de réalisation de la figure 2a.

### Description des conditions de l'étape d'hydrocraquage (2) et des séparations :

Cette description se réfère à des conditions et mises en oeuvre classiques, qui s'appliquent aussi bien à la figure 1 (art antérieur) qu'à l'invention (figures 2a et 2b).

### Charges :

Des charges très variées peuvent être traitées par les procédés d'hydrocraquage. Généralement elles contiennent au moins 10 %volume, généralement au moins 20 %volume, et souvent au moins 80 %volume de composés bouillant au-dessus de 340°C.

La charge peut être par exemple des LCO (light cycle oil - gazoles légers issus d'une unité de craquage catalytique), des distillats atmosphériques, des distillats sous vide par exemple gazoles issus de la distillation directe du brut ou d'unités de conversion telles que le FCC, le coker ou la viscoréduction, ainsi que des charges provenant d'unités d'extraction d'aromatiques des bases d'huile lubrifiante ou issues du déparaffinage au solvant des bases d'huile lubrifiante, ou encore des distillats provenant de procédés de désulfuration ou d'hydroconversion en lit fixe ou en lit bouillonnant de RAT (résidus atmosphériques) et/ou de RSV (résidus sous vide) et/ou d'huiles désasphaltées, ou encore la charge peut être une huile désasphaltée, des effluents d'une unité de Fischer-Tropsch ou encore tout mélange des charges précédemment citées. La liste ci- dessus n'est pas limitative.

En général, les charges ont un point d'ébullition T5 supérieur à 150°C (c'est-à-dire que 95 pourcent des composés présents dans la charge ont un point d'ébullition supérieur à 150°C). Dans le cas de diesel, le point T5 est généralement d'environ 150°C. Dans le cas de VGO, le T5 est généralement supérieur à 340°C, voire supérieur à 370°C. Les charges utilisables sont donc dans une large gamme de points d'ébullition. Cette gamme s'étend généralement du diesel au VGO, en passant par tous les mélanges possibles avec d'autres charges, par exemple le LCO.

La teneur en azote des charges traitées dans les procédés d'hydrocraquage est usuellement supérieure à 500 ppm poids, généralement comprise entre 500 et 10000 ppm poids, de manière plus générale comprise entre 700 et 4500 ppm poids et de manière encore plus générale comprise entre 800 et 4500 ppm poids.

La teneur en soufre des charges traitées dans les procédés d'hydrocraquage est usuellement comprise entre 0,01 et 5 % poids, de manière générale comprise entre 0,2 et 4 % poids et de manière encore plus générale entre 0,5 et 3 % poids. La charge peut éventuellement contenir des métaux. La teneur cumulée en nickel et vanadium des charges traitées dans les procédés d'hydrocraquage est de préférence inférieure à 10 ppm poids, de manière préférée inférieure à 5 ppm poids et de manière encore plus préférée inférieure à 2 ppm poids. La teneur en asphaltènes est généralement inférieure à 3000 ppm poids, de manière préférée inférieure à 1000 ppm poids, de manière encore plus préférée inférieure à 300 ppm poids.

### Lits de garde :

Dans le cas où la charge contient des composés de type résines et/ou asphaltènes, il est avantageux de faire passer au préalable la charge sur un lit de catalyseur ou d'adsorbant différent du catalyseur d'hydrocraquage ou d'hydrotraitement. Les catalyseurs ou lits de garde utilisés ont la forme de sphères ou d'extrudés. Toute autre forme peut être utilisée. Parmi les formes particulières possibles sans que cette liste soit limitative: les cylindres creux, les anneaux creux, les anneaux de Raschig, les cylindres creux dentelés, les cylindres creux crénelés, les roues de charrettes dites pentaring, les cylindres a multiples trous, etc. Ces catalyseurs peuvent avoir été imprégnés par une phase active ou non. De manière préférée, les catalyseurs sont imprégnés par une phase hydro-déshydrogénante. De manière très préférée, la phase CoMo ou NiMo est utilisée. Ces catalyseurs peuvent présenter de la macroporosité.

### Conditions opératoires :

Les conditions opératoires telles que température, pression, taux de recyclage d'hydrogène, vitesse spatiale horaire, pourront être très variables en fonction de la nature de la charge, de la qualité des produits désirés et des installations dont dispose le raffineur. Le catalyseur d'hydrocraquage/hydroconversion ou hydrotraitement est généralement mis en contact, en présence d'hydrogène, avec les charges décrites précédemment, à une température supérieure à 200 °C, souvent comprise entre 250 et 480°C, avantageusement comprise entre 320 et 450°C, de préférence entre 330 et 435°C, sous une pression supérieure à 1 MPa, souvent comprise entre 2 et 25 MPa, de manière préférée entre 3 et 20 MPa, la vitesse spatiale étant comprise entre 0,1 et 20h⁻¹ et de préférence comprise entre 0,1 et 6h⁻¹, de manière plus préférée comprise entre 0,2 et 3h⁻¹, et la quantité d'hydrogène introduite est telle que le rapport volumique litre d'hydrogène / litre d'hydrocarbure soit compris entre 80 et 5000NI/l et le plus souvent comprise entre 100 et 3000 NI/l.

Ces conditions opératoires utilisées dans les procédés d'hydrocraquage permettent généralement d'atteindre des conversions par passe, en produits convertis (i.e. à points d'ébullition inférieurs au point de coupe résidu) supérieures à 15 % et de manière encore plus préférée comprises entre 20 % et 95 %.

### Les principaux objectifs :

L'invention est utilisable pour tous les hydrocraqueurs, à savoir :
- hydrocraqueur maxi-naphta avec un point de coupe résidu généralement entre 150°C et 190°C, de préférence entre 160°C et 190°C, et le plus souvent de 170°C-180°C
- hydrocraqueur maxi-kérosène avec un point de coupe résidu généralement entre 240°C et 290°C, et le plus souvent de 260°C-280°C
- hydrocraqueur maxi diesel avec un point de coupe résidu généralement entre 340°C et 385°C, et le plus souvent de 360°C-380°C.

### Modes de mise en oeuvre :

Les procédés d'hydrocraquage/hydroconversion mettant en oeuvre les catalyseurs selon l'invention couvrent les domaines de pression et de conversion allant de l'hydrocraquage doux à l'hydrocraquage haute pression.

On entend par hydrocraquage doux, un hydrocraquage conduisant à des conversions modérées, généralement inférieures à 40 percent, et fonctionnant à basse pression, généralement entre 2 MPa et 9 MPa. Le catalyseur d'hydrocraquage peut être utilisé seul, en un seul ou plusieurs lits catalytiques en lit fixe, dans un ou plusieurs réacteurs, dans un schéma d'hydrocraquage dit en une étape, avec ou sans recyclage liquide de la fraction non convertie, éventuellement en association avec un catalyseur d'hydroraffinage situé en amont du catalyseur d'hydrocraquage.

L'hydrocraquage peut être opéré à haute pression (d'au moins 10MPa).

L'hydrocraquage peut selon une première variante être opéré selon un schéma d'hydrocraquage dit en deux étapes avec séparation intermédiaire entre les deux zones réactionnelles, dans une étape donnée, le catalyseur d'hydrocraquage peut être utilisé dans l'un ou dans les deux réacteurs en association ou non avec un catalyseur d'hydroraffinage situé en amont du catalyseur d'hydrocraquage.

L'hydrocraquage peut être opéré selon une deuxième variante, dite en une étape. Cette variante comprend généralement en premier lieu un hydroraffinage poussé qui a pour but de réaliser une hydrodéazotation et une hydrodésulfuration poussées de la charge avant que celle-ci ne soit envoyée sur le catalyseur d'hydrocraquage proprement dit, en particulier dans le cas où celui-ci comporte une zéolithe. Cet hydroraffinage poussé de la charge n'entraine qu'une conversion limitée de cette charge en fractions plus légères. La conversion, qui reste insuffisante, doit donc être complétée sur le catalyseur d'hydrocraquage plus actif.

La section d'hydrocraquage peut contenir un ou plusieurs lits de catalyseurs identiques ou différents. Lorsque les produits préférés sont les distillats moyens, on utilise des solides basiques amorphes, par exemple de l'alumine ou des silices-alumines ou des zéolithes basiques, éventuellement additionnées d'au moins un métal hydrogénant du groupe VIII et de préférence également additionnées d'au moins un métal du groupe VIB. Ces zéolithes basiques sont composées de silice, d'alumine, et d'un ou plusieurs cations échangeables tels que du sodium, du magnésium, du calcium ou des terres rares.

Lorsque l'essence est le produit majoritairement recherché, le catalyseur est généralement composé d'une zéolithe cristallisée sur laquelle on dépose de faibles quantités d'un métal du groupe VIII, et également de manière plus préférée d'un métal du groupe VIB.

Les zéolithes utilisables sont naturelles ou synthétiques et peuvent être par exemple choisies parmi les zéolithes X, Y ou L, la faujasite, la mordénite, l'érionite ou la chabasite.

L'hydrocraquage peut être opéré dans un seul ou plusieurs réacteurs en lit bouillonnant, avec ou sans recyclage liquide de la fraction non convertie, éventuellement en association avec un catalyseur d'hydroraffinage situé dans un réacteur en lit fixe ou en lit bouillonnant en amont du catalyseur d'hydrocraquage. Le lit bouillonnant s'opère avec retrait de catalyseur usé et ajout journalier de catalyseur neuf afin de conserver une activité du catalyseur stable.

### Séparation liquide/gaz (4) :

Le séparateur (4) réalise la séparation du liquide et du gaz présents dans l'effluent sortant de l'unité d'hydrocraquage. Tout type de séparateur permettant cette séparation peut être utilisée, par exemple un ballon de flash, un stripper, voire une simple colonne à distiller.

### Fractionnement (12)

La section de fractionnement est généralement constituée d'une ou plusieurs colonnes comprenant plusieurs plateaux et/ou garnissages internes qui peuvent être opérées de préférence à contrecourant. Ces colonnes sont habituellement strippées à la vapeur et comprennent un rebouilleur afin de faciliter la vaporisation. Elle permet de séparer le sulfure d'hydrogène (H2S) et les composant légers (méthane, éthane, propane, butane...) des effluents, ainsi que les coupes hydrocarbonées présentant des points d'ébullition dans le domaine des essences, du kérosène, du gasoil et une fraction lourde récupérée en fond de colonne dont tout ou partie peut être recyclée à la section d'hydrocraquage.

### EXEMPLES :

### Exemple 1 : art antérieur

Cet exemple est basé sur la configuration de la figure 1. Deux échantillons provenant d'une unité industrielle en opération, basée sur la configuration de la figure 1 ont été analysés. Les propriétés sont reportées dans le tableau 1 ci-après.

Il est à noter que compte tenu de la configuration, les flux (15a), (16), (18) et (23) ont exactement les mêmes propriétés.

Le fractionnement du flux (11) dans la colonne (12) a été simulé par programmation via le logiciel PRO/II version 8.3.3, commercialisé par la société SimSci. Les propriétés physiques et analytiques des flux résultants ont été simulées et confrontées aux propriétés physiques et analytiques des échantillons réels.

Les conditions opératoires de la colonne utilisées pour la simulation sont reportées dans le tableau 2 ci-après.

**Tableau 1 : propriété des flux selon le schéma de la figure 1**

| **Configuration** | | **Flux de la** **Figure 1** | | | |
|---|---|---|---|---|---|
| **Numéro de Flux** | | 11 | 15a | 18 | 16 |
| **Rendement** | %pds | 100 | 42 | 39,5 | 2,5 |
| **Quantité de diesel dans le flux** | %pds | 64,0 | 10,9 | 10,9 | 10,9 |
| **densité** | | 0,805 | 0,828 | 0,828 | 0,828 |

| **HPNA** | | | | | |
|---|---|---|---|---|---|
| Coronène | ppm pds | 209 | 497 | 497 | 497 |
| Dibenzo(e,ghi)pérylène | ppm pds | 33 | 78 | 78 | 78 |
| Naphtho[8.2,1 abc] coronène | ppm pds | 81 | 192 | 192 | 192 |
| Ovalene | ppm pds | 57 | 135 | 135 | 135 |
| **Total HPNA** | **ppm pds** | **378** | **902** | **902** | **902** |
| | | | | | |

| **TBP, %pds** | | | | | |
|---|---|---|---|---|---|
| Point d'ébullition Initial | °C | 128 | 200 | 200 | 200 |
| 10% | °C | 200 | 368 | 368 | 368 |
| 50% | °C | 326 | 402 | 402 | 402 |
| 90% | °C | 440 | 477 | 477 | 477 |
| Point D'ébullition final | °C | 524 | 524 | 524 | 524 |

| | | | | | |
|---|---|---|---|---|---|
| 1 : Densité relative SG= ρ_{échantillon} à 20°C/ρ_{H20} à 4°C où p est la densité exprimée en g/cm³ | | | | | |

A partir des propriétés du flux 11 d'entrée de la colonne de fractionnement (voir tableau 1), la simulation PRO/II a pu établir les propriétés du flux 15 de sortie de la colonne de fractionnement, notamment la répartition en HPNA a pu être modélisée.

**Tableau 2 : Conditions opératoires de la colonne**

| *Conditions opératoires du fractionnement* | | *Figure 1* |
|---|---|---|
| Pression haut de colonne | barg | 1,0 |
| Pression bas de colonne | barg | 1,5 |
| Température Charge entrée | °C | 377 |
| Nombre de plateaux théoriques | | 34 |
| Débit de vapeur de stripage | kg de vapeur/tonne de charge | 17 |

Sur la base de ces résultats, les configurations de l'invention ont été simulées. Les résultats sont exposés ci-dessous pour chaque configuration.

### Exemple 2 : Configuration 2a

Le tableau 3 ci-dessous donne les caractéristiques des flux 11, 16 et 18 dans la configuration 2a issu de la simulation PRO/II. Les conditions opératoires utilisées pour la simulation sont reportées tableau 4.

**Tableau 3 : propriété des flux selon le schéma de la figure 2a**

| **Configuration** | **Flux de la** **Figure 2a** | | |
|---|---|---|---|
| **Numéro de Flux** | 11 entrée | 18 recyclage liquide | 16 purge |
| **Rendement** | 100 | 39,5 | 2,5 |
| **Quantité de diesel dans le flux** | 64,0 | 14,8 | 5,1 |
| **densité** | 0,805 | 0,8275 | 0,832 |

| **HPNA** | | | |
|---|---|---|---|
| Coronène | 209 | 422 | 2192 |
| Dibenzo(e, ghi)pérylène | 33 | 96 | 314 |
| Naphtho [8.2.1 abc] coronène | 81 | 114 | 895 |
| Ovalène | 57 | 70 | 640 |
| **Total HPNA** | **378** | **702** | **4041** |
| | | | |

| **TBP, %pds** | | | |
|---|---|---|---|
| Point d'ébullition Initial | 128 | 79 | 251 |
| 10% | 200 | 362 | 389 |
| 50% | 326 | 401 | 425 |
| 90% | 440 | 476 | 505 |
| Point d'ébullition final | 524 | 524 | 524 |

| | | | |
|---|---|---|---|
| 1 : Densité relative SG= ρ_{échantillon} à 20°C/ρ_{H20} à 4°C où p est la densité exprimée en g/cm³ | | | |

**Tableau 4 : Conditions opératoires de la colonne**

| *Conditions opératoires du fractionnement* | | *Figure 2a* |
|---|---|---|
| Pression haut de colonne | barg | 1,0 |
| Pression bas de colonne | barg | 1,5 |
| Température Charge entrée | °C | 377 |
| Nombre de plateaux théoriques | | 34 |
| Débit de vapeur de stripage | kg de vapeur/tonne de charge | 17 |

Par rapport à la configuration de la figure 1, la configuration 2a permet de maximiser la quantité d'HPNA (4041 ppm poids à comparer avec 902 ppm poids selon la configuration 1) dans la fraction non convertie (résidu) qui est purgée via la ligne 16. Conjointement la quantité d'HPNA est minimisée dans le flux qui repart à la section réactionnelle via la ligne 18 : 702 ppm poids (pds) à comparer avec 902 ppm poids de la configuration 1, ce qui réduit la quantité d'HPNA de 22,2 %.

D'autre part, la proportion de HPNA lourd (Naphto [8,2,1 abc] coronène + Ovalène) par rapport à la quantité d'HPNA total dans le flux (18) qui repart à la section réactionnelle est beaucoup plus faible pour la configuration 2a (26,3 %) que pour la configuration 1 (36,3 %). Ce qui indique que non seulement il y a moins d'HPNA total dans le flux qui retourne à la section réactionnelle via la ligne 18, mais en plus que la proportion d'HPNA lourd les plus réfractaires et empoisonnants (Naphto [8.2.1 abc] coronène + Ovalène) est plus faible.

### Exemple 3 : Configuration 2b

Le tableau 5 ci-dessous donne les caractéristiques des flux 11, 16 et 18 dans la configuration 2b issu de la simulation PRO/II. Les conditions opératoires utilisées pour la simulation sont reportées tableau 6.

**Tableau 5 : propriété des flux selon le schéma de la figure 2b**

| **Configuration** | **Flux de la** **Figure 2b** | | |
|---|---|---|---|
| **Numéro de Flux** | 11 entrée | 18 recyclage Liquide | 16 purge |
| **Rendement** | 100 | 39,5 | 2,5 |
| **Quantité de diesel dans le flux** | 64,0 | 6,6 | 5,7 |
| **densité** | 0,805 | 0,8291 | 0,8313 |

| **HPNA** | | | |
|---|---|---|---|
| Coronène | 209 | 388 | 2500 |
| Dibenzo(e, ghi) pérylène | 33 | 78 | 375 |
| Naphtho [8.2.1 abc] coronène | 81 | 122 | 994 |
| Ovalene | 57 | 80 | 704 |
| **Total HPNA** | **378** | **668** | **4573** |
| | | | |

| **TBP, %pds** | | | |
|---|---|---|---|
| Point d'ébullition Initial | 128 | 217 | 210 |
| 10% | 200 | 384 | 380 |
| 50% | 326 | 401 | 421 |
| 90% | 440 | 476 | 504 |
| Point d'ébullition final | 524 | 524 | 524 |

| | | | |
|---|---|---|---|
| 1 : Densité relative SG= ρ_{échantillon} à 20°C/ρ_{H20} à 4°C où p est la densité exprimée en g/cm³ | | | |

**Tableau 6 : conditions opératoires de la colonne**

| *Conditions opératoires du fractionnement* | | *Figure 2b* |
|---|---|---|
| Pression haut de colonne | barg | 1,0 |
| Pression bas de colonne | barg | 1,5 |
| Température Charge entrée | °C | 377 |
| Nombre de plateaux théoriques | | 34 |
| Débit de vapeur de stripage | kg de vapeur/ton de charge | 17 |

| *Conditions opératoires du stripeur latéral* | | |
|---|---|---|
| Pression haut de colonne | barg | 1,4 |
| Pression bas de colonne | barg | 1,5 |
| Nombre de plateaux théoriques | | 6 |
| Débit de vapeur de stripage | kg de vapeur/t de charge | 28 |

Par rapport à la configuration de la figure 1, la configuration 2b permet de maximiser la quantité d'HPNA : 4573 ppm poids à comparer avec 902 ppm poids de la configuration 1) dans la fraction non convertie (résidu) qui est purgée via la ligne (16). Conjointement la quantité d'HPNA est minimisée dans le flux qui repart à la section réactionnelle via la ligne (18) : 668 ppm poids à comparer avec 902 ppm poids de la configuration 1, ce qui réduit la quantité d'HPNA de 25,9 %.

D'autre part, la proportion de HPNA lourd les plus réfractaires et empoisonnants (Naphto[8,2,1 abc] coronène + Ovalène) par rapport à la quantité d'HPNA total dans le flux (18) qui repart à la section réactionnelle est plus faible pour la configuration 2b (30,3 %) que pour la configuration 1 (36,3 %). Ce qui indique que non seulement il y a moins d'HPNA total dans le flux qui retourne à la section réactionnelle via la ligne (18), mais en plus que la proportion d'HPNA lourd (Naphto [8,2,1 abc] coronène + Ovalène) est plus faible.

Cette configuration permet également de minimiser la quantité de diesel qui est renvoyée à la section réactionnelle via la ligne 18 puisque la quantité de diesel incluse dans le flux qui est renvoyée à la section réactionnelle via la ligne 18 est seulement de 6,6 % poids à comparer avec 10,9 % poids dans la configuration 1.

## Revendications

1. Procédé d'hydrocraquage d'une charge pétrolière comprenant au moins 10% volume de composés bouillant au-dessus de 340°C, comprenant une étape d'hydrocraquage, éventuellement suivie d'une séparation des gaz de l'effluent hydrocraqué, puis d'une étape de fractionnement dudit effluent qui sépare au moins un distillat et un résidu, ledit résidu étant en partie recyclé vers l'étape d'hydrocraquage et une autre partie du résidu étant purgée, ladite étape de fractionnement comporte une distillation dans une colonne munie de plateaux, colonne dans laquelle
- ledit effluent au moins partiellement vaporisé alimente la colonne sur un plateau d'alimentation,
- ledit distillat est soutiré au niveau d'un plateau de soutirage,
- ledit résidu est évacué à un point d'évacuation,
- et, éventuellement, un gaz de stripage est injecté à un point d'injection situé en-dessous du plateau d'alimentation,
procédé dans lequel
- il est soutiré de la colonne une partie du flux présent au niveau d'au moins un plateau situé entre le plateau d'alimentation et le plateau de soutirage de la fraction distillat la plus lourde, ledit au moins un plateau étant situé au-dessus dudit plateau d'alimentation et en-dessous dudit plateau de soutirage de la fraction distillat la plus lourde,
- et une partie dudit flux soutiré est recyclée dans la colonne, directement ou après une éventuelle séparation du liquide,
- et l'autre partie dudit flux soutiré est recyclée dans l'étape d'hydrocraquage directement ou après une éventuelle séparation des gaz.

2. Procédé selon la revendication 1 dans lequel ledit flux qui est soutiré est présent au niveau d'un plateau situé au-dessus du plateau d'alimentation et proche dudit plateau d'alimentation, et de préférence ledit flux qui est soutiré est présent au niveau du plateau le plus proche du plateau d'alimentation.

3. Procédé selon l'une des revendications précédentes dans lequel ledit flux soutiré présente une concentration en HPNA inférieure à 500 ppm poids, préférentiellement inférieure à 350 ppm poids

4. Procédé selon l'une des revendications précédentes dans lequel ledit flux soutiré présente une proportion d'au moins 70%poids en hydrocarbures non convertis, préférentiellement d'au moins 80%poids en hydrocarbures non convertis

5. Procédé selon l'une des revendications 1 à 3 dans lequel une partie dudit flux soutiré est recyclée directement dans la colonne, l'autre partie du flux soutiré est recyclée, directement ou après une éventuelle séparation des gaz, vers l'étape d'hydrocraquage.

6. Procédé selon l'une des revendications précédentes dans lequel ledit flux soutiré est recyclé dans la colonne au-dessous du plateau d'alimentation et, lorsqu'il existe, au-dessus du point d'injection du gaz de stripage.

7. Procédé selon la revendication 6 dans lequel ledit flux soutiré est recyclé dans la colonne au niveau d'un plateau proche dudit plateau d'alimentation, et de préférence au niveau du plateau le plus proche dudit plateau d'alimentation.

8. Procédé selon l'une des revendications 1 à 3 dans lequel tout ou partie, et de préférence la totalité, dudit flux soutiré est strippé dans une étape de stripage externe par un gaz de stripage, et tout ou partie, et de préférence la totalité, de l'effluent gazeux séparé est recyclé dans la colonne au-dessus du plateau duquel le flux a été soutiré.

9. Procédé selon la revendication 8 dans lequel l'effluent gazeux est recyclé dans la colonne au niveau du plateau le plus proche du plateau duquel le flux a été soutiré.

10. Procédé selon l'une des revendications 8 à 9 dans lequel tout ou partie, et de préférence la totalité, de l'effluent liquide séparé dans ladite étape de stripage est recyclé directement dans l'étape d'hydrocraquage.

11. Procédé selon l'une des revendications précédentes dans lequel un gaz de stripage est injecté dans l'étape de fractionnement.

12. Procédé selon l'une des revendications précédentes dans lequel le gaz de stripage injecté dans l'étape de stripage externe et, si elle est présente, dans l'étape de fractionnement est de la vapeur d'eau, de préférence à une pression de 0,2-1,5MPa.

13. Procédé selon l'une des revendications précédentes ne comportant pas de recyclage du résidu dans ladite colonne de fractionnement.

14. Installation comprenant :
- une section d'hydrocraquage (2) munie d'une ligne (1) d'entrée de la charge et d'une ligne (8) d'entrée de l'hydrogène,
- éventuellement suivie d'une zone de séparation (4) de l'effluent (ligne 3) pour séparer une fraction gazeuse (ligne 5),
- suivie d'une section de fractionnement (12) comprenant au moins une colonne de distillation munie de plateaux, ladite colonne comportant :
- au moins une ligne (11) d'entrée de l'effluent hydrocraqué au moins partiellement vaporisé sur au moins un plateau d'alimentation
- au moins une ligne (14) pour le soutirage d'au moins un distillat au niveau d'un plateau de soutirage,
- au moins une ligne (15a) d'évacuation du résidu,
- au moins une ligne (23,18) pour le recyclage d'une partie du résidu dans l'étape d'hydrocraquage,
- et comprenant éventuellement au moins une ligne (19) pour l'injection d'un gaz de stripage, le point d'injection étant situé en-dessous du plateau d'alimentation,
installation comprenant en outre :
- au moins une ligne (20) pour le soutirage d'une partie du flux présent au niveau d'au moins un plateau situé entre ledit plateau d'alimentation et le plateau de soutirage de la fraction distillat la plus lourde, ledit au moins un plateau étant situé au-dessus dudit plateau d'alimentation et en-dessous dudit plateau de soutirage de la fraction distillat la plus lourde,
- et une ligne de recyclage dans ladite colonne, directement (ligne 22a) ou après une éventuelle séparation du liquide (ligne 22b), d'une partie dudit flux soutiré,
- et une ligne de recyclage dans la section d'hydrocraquage de l'autre partie dudit flux soutiré, directement (ligne 17a) ou après une éventuelle séparation des gaz (ligne 17b).

15. Installation selon la revendication 14 dans laquelle le plateau de soutirage dudit flux de la ligne (20) est positionné au niveau d'un plateau proche du plateau d'alimentation, et de préférence au niveau du plateau le plus proche du plateau d'alimentation.

16. Installation selon l'une des revendications 14 ou 15 ne comportant pas de traitement du flux soutiré avant recyclage dans la colonne.

17. Installation selon l'une des revendications 14 ou 15 ne comportant pas de traitement du flux soutiré avant recyclage dans la colonne, ladite installation comportant
- une ligne (22a) de recyclage directement dans la colonne d'une partie du flux soutiré, et
- une ligne de recyclage (17a) directement dans la section d'hydrocraquage (2) d'une partie du flux soutiré,
- avantageusement, la ligne (22a) débouche au niveau d'un plateau situé en-dessous du plateau d'alimentation et, lorsqu'il existe, au-dessus du point d'injection du gaz de stripage,
- de préférence, de plus, la ligne (22a) débouche au niveau du plateau le plus proche du plateau d'alimentation.

18. Installation selon l'une des revendications 14 ou 15 comportant:
- un stripeur (25) externe à la colonne, muni d'une ligne (20) d'entrée dudit flux soutiré, d'une ligne (26) d'injection du gaz de stripage, d'une ligne (22b) de sortie de la fraction gazeuse, d'une ligne (17b) de sortie de la fraction liquide,
- une ligne (22b) de recyclage de tout ou partie, et de préférence de la totalité, de ladite fraction gazeuse dans ladite colonne,
- de préférence, la ligne (22b) de recyclage débouche dans la colonne au-dessus du plateau duquel le flux a été soutiré, et de préférence au niveau du plateau le plus proche du plateau duquel le flux a été soutiré,
- une ligne (17b, 18) de recyclage de tout ou partie, et de préférence de la totalité, de ladite fraction liquide dans l'étape d'hydrocraquage,
- une ligne (16) pour la purge d'une partie du résidu,
- de préférence, il n'y a pas de ligne de recyclage de la fraction liquide, séparée à l'étape de stripage, vers la colonne de fractionnement.

19. Installation selon l'une des revendications 14 à 18 ne comportant pas de ligne de recyclage du résidu dans la colonne.

## Patentansprüche

1. Verfahren zum Hydrocracken einer Erdölcharge, umfassend mindestens 10 Vol.-% Verbindungen, die über 340 °C sieden, umfassend einen Hydrocracking-Schritt, gegebenenfalls gefolgt von einer Trennung von Gasen des hydrogecrackten Abstroms, dann einen Schritt der Fraktionierung des Abstroms, die mindestens ein Destillat und einen Rückstand trennt, wobei der Rückstand teilweise zu dem Hydrocracking-Schritt zurückgeführt wird, und ein anderer Teil des Rückstands gereinigt wird, wobei der Fraktionierungsschritt eine Destillation in einer Säule umfasst, die mit Böden versehen ist, wobei in der Säule:
- der mindestens teilweise verdampfte Abstrom der Säule auf einem Zufuhrboden zugeführt wird,
- das Destillat auf der Höhe eines Abzugsbodens abgezogen wird,
- der Rückstand an einem Abfuhrpunkt abgeführt wird,
- und gegebenenfalls ein Stripping-Gas an einem Einspritzpunkt eingespritzt wird, der unter dem Zufuhrboden angeordnet ist;
wobei in dem Verfahren:
- ein Teil des Stroms, welcher auf der Höhe mindestens eines Bodens vorhanden ist, der zwischen dem Zufuhrboden und dem Abzugsboden der schwersten Destillatfraktion angeordnet ist, von der Säule abgezogen wird, wobei der mindestens eine Boden über dem Zufuhrboden und unter dem Abzugsboden der schwerste Destillatfraktion angeordnet ist,
- und ein Teil des abgezogenen Stroms in die Säule zurückgeführt wird, direkt oder nach einer gegebenenfalls durchgeführten Abtrennung der Flüssigkeit,
- und der andere Teil des abgezogenen Stroms in den Hydrocracking-Schritt direkt oder nach einer gegebenenfalls durchgeführten Abtrennung von Gasen zurückgeführt wird.

2. Verfahren nach Anspruch 1, wobei der Strom, welcher abgezogen wird, auf der Höhe eines Bodens vorhanden ist, der über dem Zufuhrboden und nahe bei dem Zufuhrboden angeordnet ist, und vorzugsweise der Strom, welcher abgezogen wird, auf der Höhe des Bodens vorhanden ist, der dem Zufuhrboden am nächsten liegt.

3. Verfahren nach einem der vorhergehenden Ansprüche, wobei der abgezogene Strom eine Konzentration an HPNA von weniger als 500 ppm, bezogen auf das Gewicht, aufweist, vorzugsweise weniger als 350 ppm, bezogen auf das Gewicht.

4. Verfahren nach einem der vorhergehenden Ansprüche, wobei der abgezogene Strom einem Anteil von mindestens 70 Gew.-% nicht übergeführten Kohlenwasserstoffen aufweist, vorzugsweise von mindestens 80 Gew.-% nicht übergeführten Kohlenwasserstoffen.

5. Verfahren nach einem der Ansprüche 1 bis 3, wobei ein Teil des abgezogenen Stroms direkt in die Säule zurückgeführt wird, wobei der andere Teil des abgezogenen Stroms, direkt oder nach einer gegebenenfalls durchgeführten Abtrennung von Gasen, in den Hydrocracking-Schritt zurückgeführt wird.

6. Verfahren nach einem der vorhergehenden Ansprüche, wobei der abgezogene Strom in die Säule unter dem Zufuhrboden und, wenn er vorhanden ist, über dem Einspritzpunkt des Stripping-Gases zurückgeführt wird.

7. Verfahren nach Anspruch 6, wobei der abgezogene Strom in die Säule auf der Höhe eines Bodens nahe bei dem Zufuhrboden zurückgeführt wird, und vorzugsweise auf der Höhe des Bodens, der dem Zufuhrboden am nächsten liegt.

8. Verfahren nach einem der Ansprüche 1 bis 3, wobei der gesamte oder ein Teil, und vorzugsweise die Gesamtheit, des abgezogenen Stroms in einem externen Stripping-Schritt durch ein Stripping-Gas gestrippt wird, und der gesamte oder ein Teil, und vorzugsweise die Gesamtheit, des abgetrennten Gasabstroms in die Säule über dem Boden zurückgeführt wird, von dem der Strom abgezogen wurde.

9. Verfahren nach Anspruch 8, wobei der Gasabstrom in die Säule auf der Höhe des Bodens zurückgeführt wird, der dem Boden am nächsten liegt, von dem der Strom abgezogen wurde.

10. Verfahren nach einem der Ansprüche 8 bis 9, wobei der gesamte oder ein Teil, und vorzugsweise die Gesamtheit, des flüssigen Abstroms, der in dem Stripping-Schritt abgetrennt wird, direkt in den Hydrocracking-Schritt zurückgeführt wird.

11. Verfahren nach einem der vorhergehenden Ansprüche, wobei ein Stripping-Gas in dem Fraktionierungsschritt eingespritzt wird.

12. Verfahren nach einem der vorhergehenden Ansprüche, wobei das Stripping-Gas, das in dem externen Stripping-Schritt eingespritzt wird, und, wenn er vorhanden ist, in dem Fraktionierungsschritt Wasserdampf ist, vorzugsweise bei einem Druck von 0,2 bis 1,5 MPa.

13. Verfahren nach einem der vorhergehenden Ansprüche, welches keine Rückführung des Rückstands in die Fraktionierungssäule umfasst.

14. Anlage, umfassend:
- eine Hydrocracking-Sektion (2), die mit einer Eingangsleitung (1) der Charge und einer Eingangsleitung (8) von Wasserstoff versehen ist,
- gegebenenfalls gefolgt von einer Trennzone (4) des Abstroms (Leitung 3) zur Abtrennung einer gasförmigen Fraktion (Leitung 5),
- gefolgt von einer Fraktionierungssektion (12), umfassend mindestens eine Destillationssäule, die mit Böden versehen ist, wobei die Säule umfasst:
- mindestens eine Eingangsleitung (11) des Hydrocracking-Abstroms, der mindestens teilweise auf mindestens einem Zufuhrboden verdampft wird,
- mindestens eine Leitung (14) zum Abziehen mindestens eines Destillats auf der Höhe eines Abzugsbodens,
- mindestens eine Leitung (15a) zum Abführen des Rückstands,
- mindestens eine Leitung (23, 18) zum Zurückführen eines Teils des Rückstands in den Hydrocracking-Schritt,
- und gegebenenfalls umfassend mindestens eine Leitung (19) zum Einspritzen eines Stripping-Gases, wobei der Einspritzpunkt unter dem Zufuhrboden angeordnet ist;
wobei die Anlage außerdem umfasst:
- mindestens eine Leitung (20) zum Abziehen eines Teils des Stroms, welcher auf der Höhe mindestens eines Bodens vorhanden ist, der zwischen dem Zufuhr-boden und dem Abzugsboden der schwersten Destillatfraktion angeordnet ist, wobei der mindestens eine Boden über dem Zufuhrboden und unter dem Abzugsboden der schwersten Destillatfraktion angeordnet ist,
- und eine Leitung zum Zurückführen eines Teils des abgezogenen Stroms in die Säule, direkt (Leitung 22a) oder nach einer gegebenenfalls durchgeführten Abtrennung der Flüssigkeit (Leitung 22b),
- und eine Leitung zum Zurückführen des anderen Teils des abgezogenen Stroms in die Hydrocracking-Sektion, direkt (Leitung 17a) oder nach einer gegebenenfalls durchgeführten Abtrennung von Gasen (Leitung 17b).

15. Anlage nach Anspruch 14, wobei der Abzugsboden des Stroms der Leitung (20) auf der Höhe eines Boden positioniert ist, der nahe bei dem Zufuhrboden liegt, und vorzugsweise auf der Höhe des Bodens, der dem Zufuhrboden am nächsten liegt.

16. Anlage nach einem der Ansprüche 14 oder 15, welche keine Behandlung des abgezogenen Stroms vor dem Zurückführen in die Säule umfasst.

17. Anlage nach einem der Ansprüche 14 oder 15, welche keine Behandlung des abgezogenen Stroms vor dem Zurückführen in die Säule umfasst, wobei die Anlage umfasst:
- eine Leitung (22a) zum Zurückführen eines Teils des abgezogenen Stroms direkt in die Säule, und
- eine Leitung (17a) zum Zurückführen eines Teils des abgezogenen Stroms direkt in die Hydrocracking-Sektion (2),
- wobei vorteilhaft die Leitung (22a) auf der Höhe eines Bodens einmündet, der unter dem Zufuhrboden und, wenn er vorhanden ist, über dem Einspritzpunkt des Stripping-Gases angeordnet ist,
- wobei außerdem vorteilhaft die Leitung (22a) auf der Höhe des Bodens einmündet, der dem Zufuhrboden am nächsten liegt.

18. Anlage nach einem der Ansprüche 14 oder 15, umfassend:
- einen Stripper (25) extern von der Säule, der mit einer Eingangsleitung (20) des abgezogenen Stroms, mit einer Leitung (26) zum Einspritzen des Stripping-Gases, mit einer Ausgangsleitung (22b) der gasförmigen Fraktion, und mit einer Ausgangsleitung (17b) der flüssigen Fraktion versehen ist,
- eine Leitung (22b) zum Zurückführen der gesamten oder eines Teils, und vorzugsweise der Gesamtheit, der gasförmigen Fraktion in die Säule,
- wobei vorzugsweise die Rückführleitung (22b) in die Säule auf der Höhe des Bodens einmündet, von dem der Strom abgezogen wurde, und vorzugsweise auf der Höhe des Bodens, der dem Boden am nächsten liegt, von dem der Strom abgezogen wurde,
- eine Leitung (17b, 18) zum Zurückführen der gesamten oder eines Teils, und vorzugsweise der Gesamtheit, der flüssigen Fraktion in den Hydrocracking-Schritt,
- eine Leitung (16) zum Reinigen eines Teils des Rückstands,
- wobei vorzugsweise keine Leitung zum Zurückführen der flüssigen Fraktion, getrennt von dem Stripping-Schritt, in die Fraktionierungssäule vorhanden ist.

19. Anlage nach einem der Ansprüche 14 bis 18, welche keine Leitung zum Zurückführen des Rückstands in die Säule umfasst.

## Claims

1. A process for hydrocracking an oil feed comprising at least 10% by volume of compounds boiling above 340°C, comprising a hydrocracking step, optionally followed by a separation of the gases from the hydrocracked effluent, then a step for fractionation of said effluent, which separates at least one distillate and a residue, a portion of said residue being recycled to the hydrocracking step and another portion of the residue being purged, said fractionation step comprising a distillation in a column provided with plates, in which column:
• said at least partially vaporized effluent supplies the column over at least one supply plate,
• said distillate is withdrawn from the level of a withdrawal plate,
• said residue is evacuated at an evacuation point,
• and optionally, a stripping gas is injected at an injection point located below the supply plate,
in which process
• a portion of the stream present at the level of at least one plate located between the supply plate and the plate for withdrawing the heaviest distillate fraction is withdrawn from the column, said at least one plate being located above the supply plate and below said withdrawal plate of the heaviest distillate fraction and
• a portion of said withdrawn stream is recycled to the column, directly or after optional liquid separation,
• and the other portion of said withdrawn stream is recycled to the hydrocracking step directly or after optional separation of the gases.

2. The process as claimed in claim 1, in which a portion of the stream present at the level of a plate located above the supply plate and close to said supply plate, preferably at the level of the plate which is closest to the supply plate, is withdrawn from the column.

3. The process as claimed in one of the preceding claims, in which said withdrawn stream has a concentration of HPNA of less than 500 ppm by weight, preferably less than 350 ppm by weight.

4. The process as claimed in one of the preceding claims, in which said withdrawn stream has a proportion of at least 70% by weight of unconverted hydrocarbons, preferably at least 80% by weight of unconverted hydrocarbons.

5. The process as claimed in one of claims 1 to 3, in which a portion of said withdrawn stream is recycled directly to the column, the other portion of the withdrawn stream being recycled to the hydrocracking step, directly or after optional separation of the gases.

6. The process as claimed in one of the preceding claims, in which said withdrawn stream is recycled to the column above the supply plate and, when it exists, above the point for injection of the stripping gas.

7. The process as claimed in claim 6, in which said withdrawn stream is recycled to the column at the level of a plate close to said supply plate, and preferably to the level of the plate closest to said supply plate.

8. The process as claimed in one claims 1 to 3, in which all or a portion, preferably all, of said withdrawn stream is stripped in an external stripping step using a stripping gas, and all or a portion, preferably all of the separated gaseous effluent is recycled to the column above the plate from which the stream has been withdrawn.

9. The process as claimed in claim 8, in which the gaseous effluent is recycled to the column at the level of the plate closest to the plate from which the stream has been withdrawn

10. The process as claimed in claim 8 or claim 9, in which all or a portion, preferably all of the liquid effluent separated in said stripping step is recycled directly to the hydrocracking step.

11. The process as claimed in one of the preceding claims, in which a stripping gas is injected into the fractionation step.

12. The process as claimed in one of the preceding claims, in which the stripping gas injected into the external stripping step and, if it is present, into the fractionation step is steam, preferably at a pressure of 0.2-1.5 MPa.

13. The process as claimed in one of the preceding claims, which does not comprise recycling the residue to said fractionation step.

14. A facility comprising:
• a hydrocracking section (2) provided with an inlet line (1) for the feed and an inlet line (8) for hydrogen,
• optionally followed by a zone (4) for separating effluent (line 3) in order to separate a gaseous fraction (line 5),
• followed by a fractionation section (12) comprising at least one distillation column provided with plates, said column comprising:
∘ at least one line (11) for the inflow of at least partially vaporized hydrocracked effluent onto at least one supply plate,
∘ at least one line (14) for withdrawing at least one distillate from the level of a withdrawal plate,
∘ at least one line (15a) for evacuating residue,
• at least one line (23, 18) for recycling a portion of the residue to the hydrocracking step,
• and optionally comprising at least one line (19) for injecting a stripping gas, the injection point being located below the supply plate,
the facility further comprising:
• at least one line (20) for withdrawing a portion of the stream present at the level of at least one plate located between said supply plate and the plate for withdrawing the heaviest distillate fraction, said at least one plate being located above the supply plate and below said withdrawal plate of the heaviest distillate fraction
• and a line for recycling a portion of said withdrawn stream to said column, directly (line 22a) or after optional liquid separation (line 22b),
• and a line for recycling of the other portion of said withdrawn stream to the hydrocracking section, directly (line 17a) or after an optional gas separation (line 17b).

15. The facility as claimed in claim 14, in which the plate for withdrawing said stream from the line (20) is positioned at the level of a plate close to the supply plate, and preferably at the level of the plate closest to the supply plate.

16. The facility as claimed in claim 14 or claim 15, which does not comprise treatment of the stream withdrawn before recycling to the column.

17. The facility as claimed in claim 14 or claim 15, which does not comprise a treatment of the stream withdrawn before recycling to the column, said facility comprising:
• a line (22a) for recycling a portion of the withdrawn stream directly to the column, and
• a line (17a) for recycling a portion of the withdrawn stream directly to the hydrocracking section (2),
• advantageously, the line (22a) discharges at the level of a plate located below the supply plate and, if it exists, above the stripping gas injection point,
• preferably, in addition, the line (22a) discharges at the level of the lower plate closest to the supply plate.

18. The facility as claimed in claim 14 or claim 15, comprising:
• a stripper (25) external to the column, provided with an inlet line (20) for said withdrawn stream, a stripping gas injection line (26), an outlet line (22b) for the gaseous fraction, an outlet line (17b) for the liquid fraction,
• a line (22b) for recycling all or a portion, preferably all, of said gaseous fraction to said column,
• preferably, the recycling line (22b) discharges into the column above the plate from which the stream has been withdrawn, and preferably from the level of the plate closest to the plate from which the stream has been withdrawn,
• a line (17b, 18) for recycling all or a portion, preferably all, of said liquid fraction to the hydrocracking step,
• a line (16) to purge a portion of the residue,
• preferably, there is no line for recycling a liquid fraction separated in the stripping step towards the fractionation column.

19. The facility as claimed in one of claims 14 to 18, which does not comprise a line for recycling residue to the column.
